# EUROPEAN PATENT APPLICATION

(11) **EP 2 014 292 A2**
(43) Date of publication of application: **14.01.2009**
(21) Application number: 08166515.0
(22) Date of filing: 09.07.2003
(51) Int. Cl.: A61K 31/55, A61P 13/12

(54) **FENOLDOPAM FOR TREATMENT OF ACUTE RENAL FAILURE**

(62) Divisional of application: 03818024.6
(71) Applicant: FlowMedica, Inc., Fremont, CA 94538 (US)
(72) Inventor: Kesten, Randy J., Los Altos, CA 94022-2141 (US); Rosenthal, Michael H., Palo Alto, CA 94301-4033 (US); Payne, Sam G., Santa Clara, CA 95051 (US); Kramer, Andrew K., San Jose, CA 95118 (US); Pesotchinsky, Sophia, Los Altos, CA 94022 (US)
(74) Representative: Hamer, Christopher K.

(57) **Abstract**

The invention relates to the treatment of acute renal failure or acute renal insufficiency in a patient comprising the administration of fenoldopam or an analogue thereof. The medicament can be administered to one renal artery or to both renal arteries.

## Description

### CROSS-REFERENCE TO RELATED APPLICATIONS

Not Applicable

### STATEMENT REGARDING FEDERALLY SPONSORED RESEARCH OR DEVELOPMENT

Not Applicable

### INCORPORATION-BY-REFERENCE OF MATERIAL SUBMITTED ON A COMPACT DISC

Not Applicable

### BACKGROUND OF THE INVENTION

### 1. Field of Invention

This invention relates to the field of medical devices, and more particularly to a catheter configured for drug delivery.

### 2. Description of the Background Art

Acute renal failure ("ARF") is an abrupt decrease in the kidney's ability to excrete waste from a patient's blood. This change in kidney function may be attributable to many causes. A traumatic event, such as hemorrhage, gastrointestinal fluid loss, or renal fluid loss without proper fluid replacement may cause the patient to go into ARF. Patients may also become vulnerable to ARF after receiving anesthesia, surgery, or α-adrenergic agonists because of related systemic or renal vasoconstriction. Additionally, systemic vasodilation caused by anaphylaxis, and antihypertensive drugs, sepsis or drug overdose may also cause ARF because the body's natural defense is to shut down, i.e., vasoconstrict, non-essential organs such as the kidneys. Reduced cardiac output caused by cardiogenic shock, congestive heart failure, pericardial tamponade or massive pulmonary embolism creates an excess of fluid in the body, which can exacerbate congestive heart failure. For example, a reduction in blood flow and blood pressure in the kidneys due to reduced cardiac output can in turn result in the retention of excess fluid in the patient's body, leading, for example, to pulmonary and systemic edema.

Previously known methods of treating- ARF, or of treating acute renal insufficiency associated with congestive heart failure ("CHF"), involve administering drugs. However, many of these drugs, when administered in systemic doses, have undesirable side effects. Additionally, many of these drugs would not be helpful in treating other causes of ARF. While a septic shock patient with profound systemic vasodilation often has concomitant severe renal vasoconstriction, administering vasodilators to dilate the renal artery to a patient suffering from systemic vasodilation would compound the vasodilation system wide. In addition, for patients with severe CHF (e.g. those awaiting heart transplant), mechanical methods, such as hemodialysis or left ventricular assist devices, may be implemented. Mechanical treatments, such as hemodialysis, however, generally have not been used for long-term management of CHF. Such mechanical treatments would also not be help for patients with strong hearts suffering from ARF.

Intra-aortic balloon pumps (IABPs) have been suggested for use in diverting blood flow into branch arteries. One such technique involves placing an IABP in the abdominal aorta so that the balloon is situated slightly below (proximal to) the branch arteries. The balloon is selectively inflated and deflated in a counterpulsation mode so that increased pressure distal to the balloon directs a greater portion of blood flow into the branch arteries. Although the IABP method of counterpulsation may be effective for increasing coronary perfusion, it would not extend well to the renal arteries.

It would be a significant advance to provide an intra-aortic catheter for improved delivery of agents to a branch vessel such as a renal artery.

### BRIEF SUMMARY OF THE INVENTION

The invention is directed to a catheter controlling the flow of blood in a major blood vessel to a branch blood vessel, and particularly for delivering a therapeutic or diagnostic agent to the branch blood vessel with the blood flow thereto. The catheter generally comprises an elongated shaft, an expandable tubular member on a distal section of the shaft, and a radially expandable member on the expandable tubular member. Preferably, the elongated shaft has at least one lumen in fluid communication with an agent delivery port in a distal section of the shaft. The expandable tubular member is configured to extend within a major blood vessel up-stream and down-stream of a branch vessel, and as an interior passageway which is radially expandable within the major blood vessel to separate blood flow through the major blood vessel into an outer blood flow stream exterior to the tubular member and an inner blood flow stream within the interior passageway of the tubular member. Thus, the expandable tubular member provides a perfusion passageway in the major blood vessel. The radially expandable member is located down-stream of the agent delivery port and is positioned down-stream of the branch artery, and has an expanded configuration with an outer diameter larger than an outer diameter of the expanded tubular member located up-stream thereto. In the expanded configuration, the radially expandable member is configured constrict blood flow past an outer surface of the radially expandable member and direct at least part of the blood flow in the outer blood flow stream into the branch vessel, which, consequently, decreases the blood flow in the outer blood flow stream down- stream of the branch vessel. The catheter of the invention provides for delivery of an agent to a side branch vessel of a major vessel, and continuous perfusion of the major blood vessel. Another aspect of the invention is directed to methods of delivering a therapeutic or diagnostic agent to one or both kidney's of a patient.

The term proximal should be understood to mean locations on the catheter relatively closer to the operator during use of the catheter, and the term distal should be understood to mean locations on the catheter relatively further away from the operator during use of the catheter. The term up-stream should be understood to mean locations on the catheter relatively further upstream in the blood flow within the blood vessel, when the catheter is in place in the patient's blood vessel. The term down-stream should be understood to mean locations on the catheter relatively further down-stream in the blood flow within the blood vessel, when the catheter is in place in the patient's blood vessel.

The tubular member interior passageway defines a perfusion or blood pass-through lumen. The interior passageway is radially expandable, So that the tubular member can be expanded from an unexpanded configuration providing a low profile for insertion and advancement of the catheter within the patient's blood vessel, to an expanded configuration providing a desired level of perfusion within the blood vessel. The expanded interior passageway of the tubular member is sufficiently large to avoid or limit detrimental effects of occluding the blood vessel, and specifically, in one embodiment, the effects of infrarenal aortic occlusion. However, in addition to the inner blood flow stream within the inner lumen of the tubular member, the tubular member has an outer diameter in the expanded configuration along at least a section thereof which is configured to allow for an outer blood flow stream exterior to the tubular member which is at least in part directed or flowing to the branch vessel. As a result, the catheter can be used to deliver an agent from the agent delivery port into the outer blood flow stream and to the patient's branch vessel.

The tubular member can be expanded by a variety of suitable methods. In one embodiment, the tubular member is self-expanding. For example, a radially collapsed tubular member is expanded by release of a radially compressive force, as for example, by removal of a sheath of guide catheter from around the tubular member. Similarly, a wound or folded tubular member is expanded by allowing the member to unwind or unfold into the expanded tubular configuration. In another embodiment, the tubular member comprises a cylindrical inflatable member formed of a plurality of fluid-communicating wall chambers, which is inflated by directing inflation fluid into the wall chambers. In another embodiment, the tubular member has a braided structure, which is expanded by retracting a pull line to thereby shorten the length of the braided structure. In another embodiment, the tubular member is a balloon, which is expanded by directing inflation fluid into an wall chamber of the tubular member.

The radially expandable member is on a proximal or down-stream section of the tubular member, and is configured to restrict blood flow in the blood vessel. The radially expandable member has an expanded configuration with a larger outer diameter than the expanded tubular member. The radially expandable member may be a separate member secured to the tubular member as for example, where the radially expandable member is a balloon secured to an outer surface of a tubular member. Alternatively, the radially expandable member may be an integral part of the tubular member so that the tubular member and radially expandable member are a one-piece unit of the catheter, as for example, where the tubular member is a frame or braided structure having a sheath thereon and the radially expandable member is a radially enlarged section of the tubular member which expands as the tubular member expands, or where the tubular member is cone shaped and the radially expandable member is the largest diameter section of the cone shaped tubular member.

In the expanded configuration, the radially enlarged member is configured to decrease blood flow in the outer blood flow stream down-stream of the branch vessel. Thus, a relatively large concentration of agent is delivered into the branch vessel from the agent delivery port, in comparison to the amount of agent allowed to flow through the blood vessel down-stream of the branch vessel. In one embodiment, the radially expandable member has an expanded outer diameter configured to partially occlude, i.e., restrict but not completely block, the outer blood flow stream in the blood vessel. Thus, a portion of the outer blood flow through the blood vessel is allowed to flow around and down-stream of an outer surface of the radially expandable member. However, in another embodiment, the radially expandable member has an outer diameter configured to contact a wall of the blood vessel and thereby occlude the outer blood flow stream in the blood vessel down-stream of the branch vessel.

Thus, the catheter of the invention separates the blood flow through the blood vessel into an outer blood flow stream directed in part into the branch vessel having a relatively high concentration of agent, and an inner blood flow stream. The end of the tubular member positioned up-stream of the branch vessel is located up-stream of agent delivery port in the shaft, so that the inner blood flow stream within the tubular member has a relatively low amount of or no agent. Moreover, with the radially expandable member in the expanded configuration, the blood flow exterior to the tubular member down-stream of the branch vessel is decreased in comparison to the blood flow stream exterior to the tubular member up-stream of the branch vessel. As a result, the amount of agent in the outer blood flow stream directed into the branch vessel is improved.

The catheter of the invention can be used to deliver a variety of therapeutic or diagnostic agents to the patient's blood vessel. In one embodiment, vasoactive and/or renal protective agents such as Papaverine, are delivered to the renal arteries for treatment of ARF and fluid overload. Other preferred agents include Calcium-channel blockers such as nifedipine or verapamil, and fenoldapam, a dopamine DA₁ agonist. The tubular member inner lumen providing a perfusion pathway allows the catheter to be in place in the patient's blood vessel for extended periods of treatment. The period of treatment will depend on the application and the agent, but is typically about 2 to about 72 hours, preferably about 4 to about 8 hours.

It is to be appreciated, therefore, that another aspect of the invention locally delivers these drugs bilaterally into each side of the renal system via 2 renal arteries perfusing each kidney respectively. While the specific devices described are beneficial modes of such aspect, other devices, systems or specific delivery methods are contemplated.

The catheter of the invention provides improved agent delivery to a branch vessel with continuous perfusion of the major blood vessel due to the relatively large perfusion lumen in the tubular member. Thus, possible detrimental effects of infrarenal aortic occlusion are reduced or prevented. Moreover, the catheter of the invention provides a relatively large concentration of agent to the renal arteries with little loss of blood flow through the aorta to the lower limbs. The catheter configured for intra-aortic delivery of an agent provides for relatively quick, intraluminal placement of the catheter.

Another aspect of the invention is a method for enhancing renal function in a patient, where a portion of aortic blood flowing within an abdominal aorta is diverted along a flow path into the renal arteries via a number of ostia placed along the abdominal aorta wall. A volume of fluid agent adapted to enhance renal function is injected into the diverted flow path and is delivered into the renal arteries via the diverted flow path. More particularly, the injected fluid agent is delivered simultaneously and only into the renal arteries. A further aspect is to allow a second portion of aortic blood to flow along a second flow path downstream into downstream circulation while diverting the first portion of aortic blood into the renal arteries. A further beneficial embodiment is to use a fluid delivery port to deliver a volume of fluid agent into the diverted flow path. In a variation of this embodiment, the flow diverter generally comprises an adjustable wall and in a more particular embodiment, a tubular member.

Another aspect of the invention generally comprises a bi-lateral renal drug delivery system adapted to deliver a volume of fluid agent to each renal artery simultaneously and thereby perfusing each kidney.

Another aspect of the invention is a method for enhancing renal function in a patient including: diverting a portion of aortic blood flowing into a location within an abdominal aorta to flow along a diverted flow path substantially only into a plurality of renal arteries via a plurality of respective ostia having unique respective locations along the abdominal aorta wall; and injecting a volume of fluid agent into the diverted flow path within the abdominal aorta at the location. Further to this method, the injected fluid agent is delivered substantially only into the plurality of renal arteries via the diverted flow path into their respective ostia. Moreover, the fluid agent being delivered into the plurality of renal arteries is adapted to enhance renal function.

One mode of this aspect further includes locally delivering a volume of diuretic fluid agent into the plurality of renal arteries. In one embodiment of this mode, the diuretic is Furosemide or an analog or derivative thereof. In another embodiment, it is Thiazide or an analog or derivative thereof.

According to another mode, the local delivery of fluid agent into the plurality of renal arteries includes locally delivering a volume of vasopressor into the plurality of renal arteries. In one embodiment of this mode, the vasopressor is Dopamine or an analog or derivative thereof.

In another mode of this method, the fluid agent is a vasodilator.

In another mode, the fluid agent is a vasoactive fluid agent.

In another mode, the fluid agent is Papaverine.

In another mode, the fluid agent is an analog or derivative of Papaverine.

In another mode, the fluid agent is a Calcium-channel blocker.

In another mode, the fluid agent is Nifedipine.

In another mode, the fluid agent is an analog or derivative of Nifedipine.

In another mode, the fluid agent is Verapamil.

In another mode, the fluid agent is an analog or derivative of Verapamil.

In another mode, the fluid agent is Fenoldapam.

In another mode, the fluid agent is an analog or derivative of Fenoldapam into the plurality of renal arteries.

In another mode, the fluid agent is a dopamine DA₁ agonist.

According to still a further mode of the local renal drug delivery method, the fluid agent is delivered into the plurality of renal arteries for between about 2 and about 72 hours. In one embodiment, the fluid agent is delivered into the plurality of renal arteries for between about 4 and about 8 hours.

In still a further highly beneficial mode, the injected fluid agent is delivered substantially only into the plurality of renal arteries via their respective ostia simultaneously.

In another mode, the method further includes allowing a second portion of aortic blood to flow along a second flow path downstream across the plurality of renal artery ostia and into downstream circulation. This is done while diverting the portion of aortic blood into diverted flow path into the renal arteries and also while delivering the fluid agent into the plurality of renal arteries via the diverted flow path.

Another aspect of the invention is a system for enhancing renal function in a patient that includes a delivery catheter, a flow diverter, and a source of fluid agent. The delivery catheter has a proximal end portion and a distal end portion with a fluid delivery port. The flow diverter is located along the distal end portion. The delivery catheter is adapted to couple to the source of fluid agent, and also to position the distal end portion at a location within an abdominal aorta associated with a plurality of renal artery ostia having unique respective positions along the abdominal aorta wall. The flow diverter at the location is adapted to divert a portion of aortic blood flow along a diverted flow path substantially only into the plurality of renal arteries via their respective ostia. Moreover, the fluid delivery port is positioned relative to the flow diverter so as to inject a volume of the fluid agent from the source into the diverted flow path. The fluid agent is adapted to enhance renal function.

According to one mode of this aspect, the fluid agent is a diuretic. In one embodiment, the diuretic is Furosemide or an analog or derivative thereof. In another embodiment, it is Thiazide or an analog or derivative thereof.

According to another mode, the fluid agent is a vasopressor. In one embodiment, the vasopressor is Dopamine or an analog or derivative thereof.

According to still another mode, the fluid agent is a vasodilator.

According to another mode, the fluid agent is a vasoactive agent.

According to another mode, the fluid agent is Papaverine.

According to another mode, the fluid agent is an analog or derivative of Papaverine.

According to another mode, the fluid agent is a Calcium-channel blocker.

According to another mode, the fluid agent is Nifedipine.

According to another mode, the fluid agent is an analog or derivative of Nifedipine.

According to another mode, the fluid agent is Verapamil.

According to another mode, the fluid agent is an analog or derivative of Verapamil.

According to another mode, the fluid agent is Fenoldapam.

According to another mode, the fluid agent is an analog or derivative of Fenoldapam.

According to another mode, the fluid agent is a dopamine DA₁ agonist.

According to still a further mode, the delivery catheter is further adapted to allow a second portion of aortic blood to flow downstream across the plurality of renal artery ostia and into downstream circulation. This is accomplished while the flow diverter is adjusted to divert the portion of aortic blood flow along the diverted flow path, and while the fluid agent is injected into the diverted flow path.

According to yet another mode, the flow diverter includes an adjustable wall that is adjustable between a first position and a second position. In the first position the flow diverter is adapted to be delivered to the location within the abdominal aorta. In the second position the adjustable wall is adapted to divert the portion of aortic blood flow along the diverted flow path. In one embodiment, the adjustable wall is a tubular member, such that the first position is characterized as a radially collapsed condition for the tubular member, and the second position is characterized as a radially expanded condition for the tubular member. In one variation of this embodiment, the tubular member has a conical shape. In another variation, the tubular member has a frustroconical shape. In another variation, the tubular member is an inflatable member. In still a further variation, the tubular member is adapted to radially engage the abdominal aorta wall in the radially expanded condition at the location.

Another aspect of the invention is a system for treating a renal system in a patient with a renal drug delivery system coupled to a volume of fluid agent. The renal drug delivery system is adapted to deliver the volume of fluid agent bilaterally to each of two renal arteries simultaneously. The fluid agent is Papaverine, a Calcium-channel blocker, Nifedipine, Verapamil, Fenoldapam, or a dopamine DA₁ agonist. In further modes, the fluid agent includes a combination or blend of two or more of these agents. In another mode, the fluid agent includes an analog or derivative of one or more of these agents.

In one particular mode, the agent is Papaverine.

In another particular mode, the agent is a Calcium-channel blocker.

In another particular mode, the agent is Nifedipine.

In another particular mode, the agent is Verapamil.

In another particular mode, the agent is Fenoldapam.

In another particular mode, the agent is a dopamine DA₁ agonist.

Another aspect of the invention is a system for treating a renal system in a patient with a local renal drug delivery system coupled to a volume of fluid agent. The renal drug delivery system is adapted to deliver the volume of fluid agent bilaterally to each of two renal arteries simultaneously. The fluid agent includes an analog or derivative of Papaverine, Nifedipine, Verapamil, or Fenoldapam. In further modes, the fluid agent may include a combination or blend of two or more of these agents.

Another aspect of the invention is a method for treating a renal system in a patient that includes locally delivering a volume of fluid agent bilaterally into each of two renal arteries perfusing each of two kidneys substantially simultaneously. Further to this method, the fluid agent being bilaterally delivered into the renal arteries includes Papaverine, a Calcium-channel blocker, Nifedipine, Verapamil, Fenoldapam, or a dopamine DA₁ agonist. In further modes, the fluid agent may include a combination or blend of two or more of these agents. In another mode, the fluid agent includes an analog or derivative of one or more of these agents.

Another aspect of the invention is a method for treating a renal system in a patient that includes locally delivering a volume of fluid agent bilaterally into each of two renal arteries perfusing each of two kidneys substantially simultaneously. Further to this method, the fluid agent being bilaterally delivered into the renal arteries is an analog or derivative of Papaverine, a Calcium-channel blocker, Nifedipine, Verapamil, Fenoldapam, or a dopamine DA₁ agonist. In further modes, the fluid agent may include a combination or blend of two or more of these agents. In another mode, the fluid agent includes an analog or derivative of one or more of these agents.

It is to be appreciated that each of the various aspects, modes, embodiments, variations, and features herein shown and described is considered independently beneficial, either in the context of its utility for forming combinations such as for example with other elements otherwise herein described, or otherwise in its own regard, and therefore is not intended to be necessarily limited by combinations with such other elements unless specifically required. Notwithstanding the foregoing, such combinations of elements as herein shown and described are nevertheless also considered independently valuable, as would be apparent to one of ordinary skill based upon the totality of this disclosure and by reference to other available information.

These and other advantages of the invention will become more apparent from the following detailed description of the invention and the accompanying exemplary drawings.

### BRIEF DESCRIPTION OF THE SEVERAL VIEWS OF THE DRAWING(S)

FIG. 1 is an elevational view, partially in section, of a catheter which embodies features of the invention, illustrating the expandable tubular member and balloon in an unexpanded configuration.

FIG. 2 is an enlarged view, partially in phantom, of a distal section of the catheter shown in FIG. 1, with the tubular member in the expanded configuration.

FIG. 3 is an enlarged view, partially in phantom, of a distal section of the catheter shown in FIG. 2, illustrating the catheter in the patient's descending aorta proximate the renal arteries, with the balloon in the inflated configuration.

FIG. 4 is a transverse cross sectional view of the balloon shown in FIG. 3, taken along line 4-4.

FIG. 5A is a transverse cross sectional view of the balloon shown in FIG. 3, taken along line 5-5.

FIG. 5B is an another embodiment of a transverse cross sectional view of the balloon shown in FIG. 3

FIG. 6A is a transverse cross sectional view of the balloon shown in FIG. 3, taken along line 6-6.

FIG. 6B is another embodiment of a transverse cross sectional view of the balloon shown in FIG. 3, taken along line 6-6.

FIG. 7 is an enlarged view, partially in phantom, of a distal section of another embodiment having an expandable tubular member comprising a sheath covered collapsible frame.

FIG. 8 is an enlarged view, partially in phantom, of a distal section of an another embodiment having a radially expandable member comprising a radially enlarged section of the expandable tubular member.

FIG. 9A is a transverse cross sectional view of another embodiment having an expandable tubular member with a small profile wrapped configuration.

FIG. 9B is a transverse cross sectional view of the tubular member shown In FIG. 9A, illustrating the tubular member in the expanded unwrapped configuration.

FIG. 10A is a transverse cross sectional view of another embodiment having an expandable tubular member with a small profile wound configuration

FIG. 10B is a transverse cross sectional view of the tubular member shown in FIG. 10A, illustrating the tubular member in the expanded unwound configuration.

FIG. 11 is a transverse cross-sectional view of another embodiment of an expandable tubular member comprising a plurality of inflatable balloons within an outer sheath.

FIG. 12 illustrates two sheets of polymeric film as the starting material for fabricating an expandable tubular member according to one exemplary embodiment of the invention.

FIG. 13 is a perspective view of a fixture for heat fusion welding the film sheets in FIG. 12.

FIG. 14 is an elevation view of the welding fixture in FIG. 13 showing the placement of the film sheets.

FIG. 15 illustrates a cross section of two sheets of film welded by heat fusion.

FIG. 16A shows a typical pattern that would be used to weld the film sheets together to form the expandable tubular member.

FIG. 16B illustrates the welded film sheets of FIG. 16A after trimming.

FIG. 17 is a perspective view of a fixture used to configure the expandable tubular member into a tubular member.

FIG. 18 is an elevation view of FIG. 17 showing the detail of the welding method.

FIG. 19A shows a method of connecting the inflation tube to the expandable tubular member.

FIG. 19B shows the inflation tube secured to the expandable tubular member.

FIG. 20 illustrates the inflation tube entering the catheter shaft.

FIG. 21 is a transverse cross sectional view of the catheter shaft shown in FIG. 20, illustrating the position of the inflation tube.

FIG. 22 illustrates the expandable tubular member in the inflated configuration.

FIG. 23A shows a step of a folding process for the expandable tubular member shown in FIG. 22.

FIG. 23B shows another step of a folding process for the expandable tubular member shown in FIG. 22.

FIG. 23C shows another step of a folding process for the expandable tubular member in FIG. 22.

FIG. 23D shows another step of a folding process for the expandable tubular member shown in FIG. 22.

FIG. 24 shows a sheath positioned over the folded expandable tubular member shown in FIG. 23D.

FIG. 25 illustrates an enlarged view of a distal section of another embodiment having an expandable tubular member comprising a plurality of inflatable wall chambers and a balloon secured together to form the tubular member.

FIG. 26 is a transverse cross sectional view, taken along line 26-26, of the catheter shaft shown in FIG. 25.

FIG. 27 is a transverse cross sectional view, taken along line 27-27, of the expandable tubular member shown in FIG. 25.

FIG. 28 illustrates an enlarged view of a distal section of another embodiment having an expandable tubular member comprising a plurality of inflatable wall chambers, wherein the tubular member has a conical shape.

FIG. 29 is an elevational view of fused polymeric sheets used to form the tubular member, having straight seal lines forming the fluid communicating chambers.

FIG. 30 is an elevational view of fused polymeric sheets used to form the tubular member, having curved seal lines forming the fluid-communicating chambers.

FIG. 31 is a transverse cross-sectional view of a tubular member illustrated in FIG. 30.

### DETAILED DESCRIPTION OF THE INVENTION

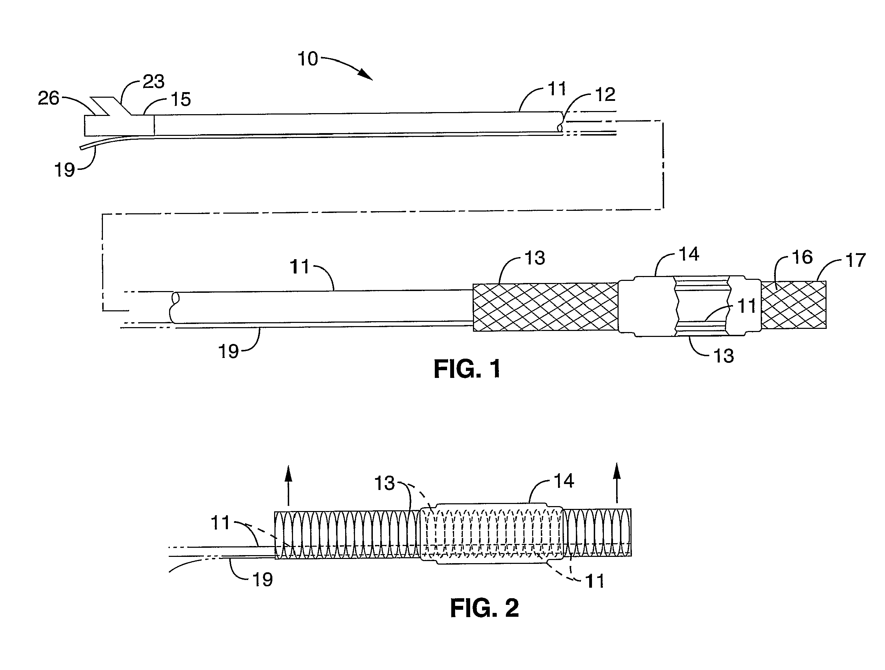

FIG. 1 illustrates a catheter 10 which embodies features of the invention, generally comprising an elongated shaft 11 having a proximal end, a distal end, and at least one lumen 12 extending therein, a tubular member 13 on a distal section of the catheter shaft 11 and a radially expandable member 14 on the tubular member 13. Adapter 15 on the proximal end of the shaft provides access to the catheter lumen. FIG. 1 illustrates the tubular member and the radially expandable member in low profile, unexpanded configurations for entry into the patient's blood vessel.

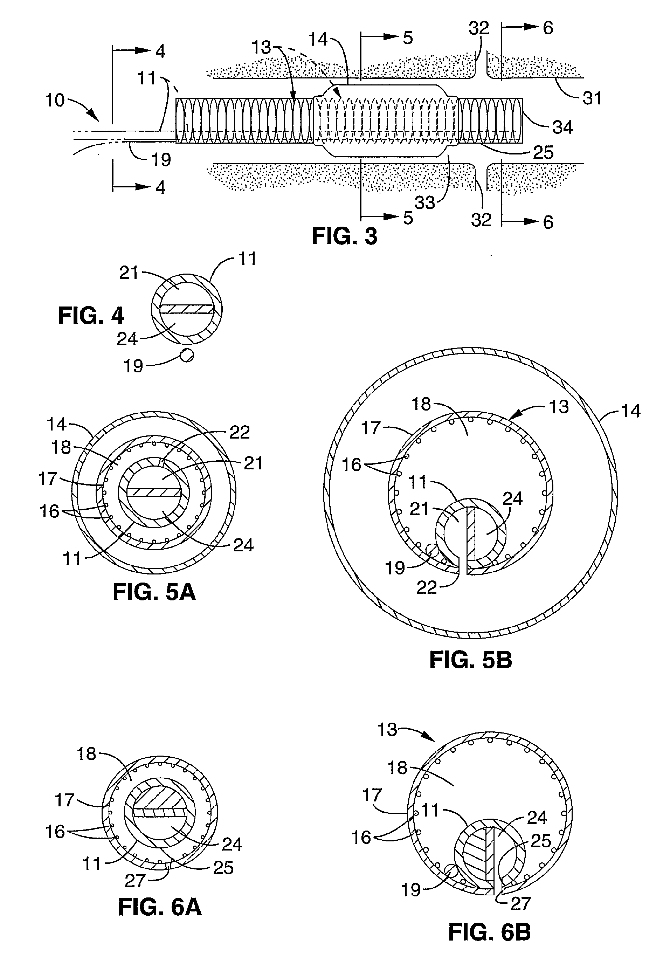

In the embodiment illustrated in FIG. 1, the radially expandable member 14 comprises an inflatable balloon. The balloon has proximal and distal ends secured to an outer surface of the tubular member 13, and an interior in fluid communication with an inflation lumen 21 (shown in FIG. 4) in the shaft 11. The balloon 14 can be formed of a variety of suitable materials typically used in the construction of catheter occlusion balloons, and in another embodiment is highly compliant and is formed of a material such as latex, polyisoprene, polyurethane, a thermoplastic elastomer such as C-Flex. In another embodiment, the balloon may be noncompliant or semi-compliant. While discussed below primarily in terms of a radially expandable member comprising a balloon, it should be understood that the radially expandable member may have a variety of suitable configurations.

In the embodiment illustrated in FIG. 1, the tubular member 13 comprises braided filaments 16, such as wire, ribbon, and the like, having a sheath 17, and having a lumen or interior passageway 18 (shown in FIG. 5) therein. A pull line 19 having a distal portion secured to the tubular member is configured to be retracted or pulled proximally to radially expand the tubular member 13. Specifically, the braided filaments 16 can reorient from a longer, smaller diameter configuration and a shorter, larger diameter configuration cause the tubular member to shorten, thereby radially expanding the tubular member 13. When the pull line 19 is not under tension, the spring force of the elastomeric material of the sheath 17 will cause the tubular body defined by the braided filaments 16 to elongate and reduce in diameter. The sheath 17 is preferably an elastomeric polymer on the braided filaments. The sheath 17 can be on an inner or outer surface of the braided filaments, or the braided filaments can be completely or partially embedded within the sheath 17. In the embodiment in which the sheath is on a surface of the filaments, the sheath is preferably secured to a surface of the filaments as for example with adhesive or heat bonding. The braided filaments 16 can be formed of a variety of suitable materials such as metals or stiff polymers. A variety of suitable polymeric materials can be used to form the sheath 17. While discussed below primarily in terms of a tubular member comprising a braided tube, it should be understood that the tubular member may have a variety of suitable configurations.

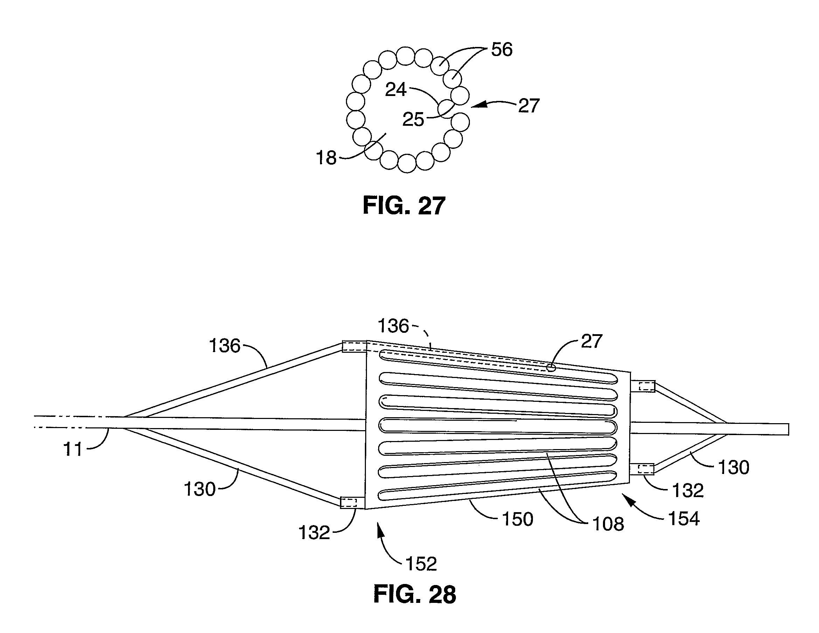

The dimensions of catheter 10 are determined largely by the size of the blood vessel(s) through which the catheter must pass, and the size of the blood vessel in which the catheter is deployed: The length of-the-tubular member 13 is typically about 50 to about 150 mm, preferably about 80 to about 120 mm. The tubular member 13 has an unexpanded outer diameter of the tubular member is typically about 1 to about 5 mm, preferably about 2 to about 4 mm, and a radially expanded outer diameter of about 40 to about 140 mm, preferably about 60 to about 120 mm. The radially expanded interior passageway 18 of the tubular member 13 is typically about 30 to about 130 mm, preferably about 50 to about 110 mm to provide sufficient perfusion. The interior passageway 18 of the tubular member 13 has a radially expanded inner diameter which is about 1000% to about 6000% larger than the unexpanded inner diameter of the passageway 18. The radially expandable member 14 has a length of about 10 to about 50 mm, preferably about 20 to about 40 mm. The expanded outer diameter of the radially expandable member 14 is about 10 to about 35 mm, preferably about 15 to about 30 mm. In an embodiment having a conically shaped tubular member (See FIG. 28), the tubular member dimensions given above should be understood to refer to the distal most (i.e., up-stream) or smaller diameter end of the conical member, unless otherwise stated. Similarly, in an embodiment in which the radially expandable member 14 comprises the larger diameter end of a conically shaped tubular member, the radially expandable member dimensions should be understood to refer to the proximal most (i.e., down-stream) or larger diameter end of the conical member.

Typically, the shaft 11 has an outer diameter of about 1 to about 5 mm. The inflation lumen 21 has an inner diameter of about 0.02 to about 0.06 mm and the agent delivery lumen has an inner diameter of about 0.01 to about 0.04 mm. The length of the catheter is about 40 to about 100 cm, preferably about 60 to about 90 cm.

FIG. 2 illustrates the tubular member 13 in the expanded configuration after retraction of the pull line 19. As best illustrated in FIG. 2, showing the distal section of the shaft 11 within the inner lumen of the tubular member 13 in dotted phantom lines, the exposed distal end of the shaft 11 is located proximal to the distal end of the expandable tubular member 13. In the embodiment illustrated in FIG. 2, the balloon 14 is in a non-expanded configuration. The section of the tubular member under the balloon is illustrated in dashed phantom lines.

FIG. 3 illustrates schematically, the expanded tubular member 13 with the balloon 14 in the expanded configuration. As best illustrated in FIG. 4, FIG. 5A and FIG. 6A showing transverse cross sections of the catheter shown in FIG. 3, taken along lines 4-4, 5-5, and 6-6, respectively, the shaft has an inflation lumen 21 extending from the proximal end of the shaft 11 to an inflation port 22 (shown in FIG. 5A) located on the shaft distal section, in fluid communication with the interior of the balloon. Arm 23 on adapter 15 (shown in FIG. 1) provides access to the inflation lumen 21, and is in fluid communication with a source of inflation fluid (not shown). The shaft also has an agent delivery lumen 24 extending from the proximal end to an agent delivery port 25 in the distal end of the shaft 11. Arm 26 on adapter 15 (shown in FIG. 1) provides access to the agent delivery lumen 24, and is in fluid communication with an agent source (not shown). The tubular member sheath 17 has an agent delivery opening 27 adjacent to the shaft agent delivery port 25, for providing a pathway for agent delivery from the lumen 24 to exterior to the tubular member 13. In the illustrated embodiment, the inflation lumen 21 and agent delivery lumen 24 are side-by-side in a multilumen shaft 11, with inflation port 22 extending through a side wall of the shaft. However, a variety of suitable configurations may be used as are conventionally used in catheter shaft design including coaxial lumens in fluid communication with side ports or ports in the distal extremity of the shaft. The agent delivery port 25 is preferably in a side wall of the shaft 11 distal section in fluid communication with the agent delivery lumen 24, however, alternatively, the agent delivery port 25 may be in the distal end of the shaft 11.

These embodiments are illustrated schematically and the relationship of the elements may be combined in various combinations and specific modes by one of ordinary skill in the art. For example, FIG. 5B and FIG. 6B illustrate more specific embodiments where multilumen shaft 11 is attached to the inner wall of tubular member 13. Inflation lumen 21 is in fluid communication through inflation port 22 and agent delivery lumen 24 is in fluid communication with blood flow 33 through agent delivery port 25 and agent delivery opening 27.

FIG. 3 illustrates the catheter 10 in a blood vessel 31, such as a descending aorta, of a patient, having branch vessels 32, such as the renal arteries, opening therein. The catheter 10 is introduced and advanced within the patient's blood vessel 31 in the low profile, unexpanded configuration illustrated in FIG. 1. The agent delivery port 25 is positioned proximate to (up-stream or inline with) the one or more branch vessels 32, and the distal end of the tubular member is preferably up-stream of the one or more branch vessels 32. The tubular member is then expanded to the expanded configuration, and, preferably, thereafter the balloon 14 is radially expanded by directing inflation fluid into the balloon interior.

Specifically, in one embodiment of a method of the invention for delivery of a therapeutic or diagnostic agent to one or more of a patient's kidneys, the catheter is introduced into the femoral artery, as for example by the Seldinger technique, preferably slidingly over a guide wire (not shown), and advanced into the descending aorta 31. Although not illustrated, the shaft may be provided with a separate guide wire lumen, or the catheter may be advanced over a guide wire in agent delivery lumen 24 adapted to slidingly receive a guide wire. Alternatively, the catheter 10 may be advanced without the use of a guide wire. The agent delivery port 25 is positioned proximate to one or both renal arteries 32, as illustrated in FIG. 3, and the tubular member 13 extends within the aorta 31 up-stream and down-stream of the renal arteries 32. The tubular member 13 is radially expanded by retracting pull line 19. The interior passageway 18 of the tubular member 13 separates blood flow through the blood vessel 31 into an outer blood flow stream 33 exterior to the tubular member 13, and an inner blood flow stream 34 within the interior passageway 18 of the tubular member 13.

The balloon 14 is expanded by directing inflation fluid into the inflation lumen 21. In the embodiment illustrated in FIG. 3, the balloon 14 is expanded to an outer diameter which does not completely occlude the patient's aorta 31. However, in another embodiment, the balloon expands into contact with the wall of the aorta 31, to an outer diameter which completely occludes the aorta 31 (not shown). Balloon 14 may have a length and elongated configuration configured to provide mechanical stability for and coaxial centering of the operative distal section of the catheter in the blood vessel 31. A stabilizing member (not shown) may be provided on an outer surface of the distal end of the tubular member 13, such as for example unfoldable arms which anchor the distal end of the catheter in the aorta 31 during delivery of agent.

A variety of suitable imaging modalities may be used to position the catheter in the desired location in the blood vessel, such as fluoroscopy, or ultrasound. For example, radiopaque markers (not shown) on the shaft may be used in positioning the balloon 14 and agent delivery port 25 at the desired location in the blood vessel 31.

A therapeutic or diagnostic agent (hereafter "agent") is delivered to the renal arteries 32 by introducing the agent into the agent delivery lumen 24 in the shaft 11, and out the agent delivery port 25. An agent delivery opening 27 in the tubular member 13 adjacent to the agent delivery port 25 provides a pathway for agent delivery from lumen 24 to external to the tubular member 13. The agent delivery port 25 is up-steam of the renal arteries 32 and proximal to the distal end of the tubular member 13. Thus, the outer blood flow stream 33 has a relatively high concentration of agent and the inner blood flow stream 34 has a relatively low concentration or no agent. Additionally, the balloon 14 in the expanded configuration restricts the flow of blood to decrease the blood flow exterior to the proximal portion of the tubular member 13 down-stream of the renal arteries 32 in comparison to the blood flow stream exterior to the distal portion of the tubular member 13 up-stream of the renal arteries 32. As a result, a relatively large amount of the agent delivered from the agent delivery port 25 is directed into the renal arteries 32, in comparison to the amount of agent which flows down-stream of the renal arteries 32 in the aorta 31. In one embodiment, the outer blood flow stream is substantial.

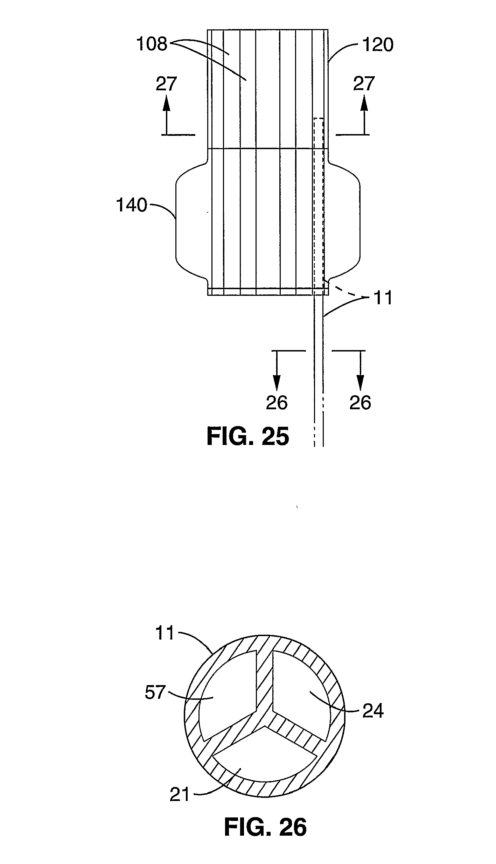

Preferably, the cross-sectional area of the inner lumen 18 of the tubular member 13 is about 4% to about 64% of the blood vessel 31 (i.e., aorta) cross-sectional area, or about 4 mm to about 16 mm for a blood vessel 31 having a 20 mm inner diameter. It should be noted that in some embodiments, the cross-sectional area of the wall of the tubular member 13 is not insignificant in relation to the cross- sectional area of the blood vessel 31. In the embodiment illustrated in FIG. 1 in which tubular member 13 comprises sheath 17 on a frame of filaments 16, this cross-sectional area is negligible. In another embodiments discussed below, such as the embodiments illustrated in FIGS. 25 and 28, the cross-sectional area of the wall of the tubular member 13 may be about 2% to about 50%, more specifically about 5% to about 20%, of the cross-sectional area of a section of the blood vessel 31 located at the up-stream most end of the catheter 10.

Additionally, the aorta has multiple branch vessels in addition to the renal arteries which effect the total flow in the aorta at a given location therein. Thus, a percentage of the blood flow that enters the abdominal aorta, i.e., past the diaphragm, is delivered in the normal rest state of circulation to the celiac trunk, the superior and inferior mesenteric arteries, and the renal arteries. Nonetheless, the flow segmentation created by the presence of the deployed catheter 10 is such that the blood flow in the outer blood flow stream 33 of a patient at rest is about 10% to about 90% of the total blood flow immediately up-stream of the up-stream or distal most end of the tubular member 13, i.e., of the total blood flow present in the section of the aorta 31 immediately adjacent to the renal arteries 32. Similarly, the blood flow in the inner blood flow stream 34 of a patient at rest is about 10% to about 90% of the total blood flow immediately up-stream of the up-stream or distal most end of the tubular member 13. The flow in the outer blood flow stream 33 is , sufficient to provide adequate kidney function, although the flow required will vary depending upon factors such as the presence of drugs which increase flow or increase the ability of the tissue to withstand ischemic conditions.

While the renal arteries 32 are illustrated directly across from one another in FIG. 3, and the method is discussed primarily in terms of delivery of agent to both renal arteries together, it should be understood that the catheter may be positioned and used- to deliver agent to the renal arteries individually, and specifically in anatomies having the renal arteries longitudinally displaced from one another. The flow of agent is then stopped. The tubular member 13 is contracted by urging the pull line 19, distally, and the balloon 14 is collapsed by removal of the inflation fluid, and the catheter removed from the patient. A variety of suitable radially expandable tubular members 13 may be used in the catheter 10 of the invention.

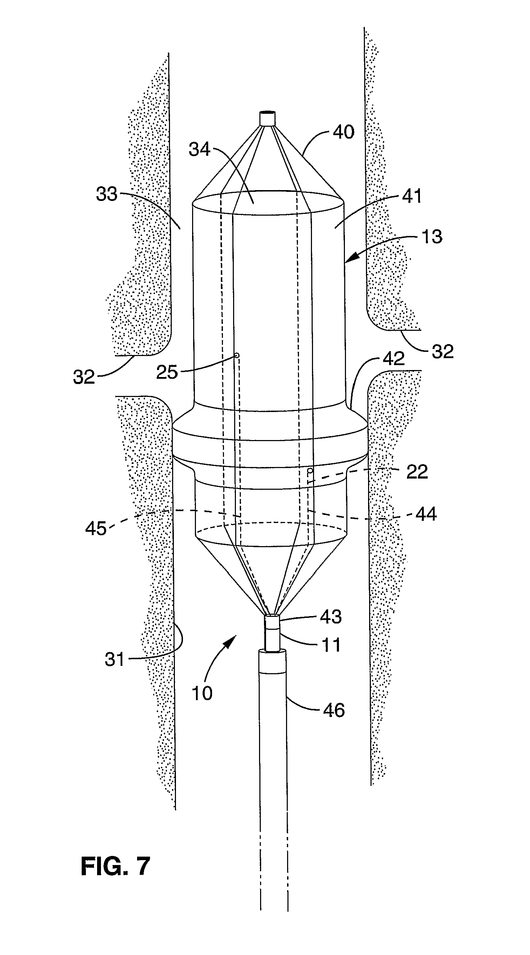

FIG. 7 illustrates another embodiment of distal end of the catheter 10 in which the tubular member 13 comprises a self- expanding frame 40 having a sheath 41 thereon. As discussed above in relation to the embodiment of FIG. 1, catheter shaft 11 defines an inflation lumen 21 and an agent delivery lumen 24, and radially expandable member comprises a balloon 42 on an outer surface of sheath 41. For ease of illustration, the balloon 42 is shown as a transparent material. In the embodiment illustrated in FIG. 7, catheter shaft 11 comprises a multilumen proximal shaft 43 defining proximal sections of the inflation lumen 21 and agent delivery lumen 24, a first distal tubular member 44 defining a distal section of inflation lumen 21 extending to inflation port 22, and a second distal tubular member 46 defining a distal section of agent delivery lumen 24 extending to agent delivery port 25. First tubular member 44 extends distally from the distal end of the proximal section of the inflation lumen 21 in the multilumen proximal shaft. Similarly, second tubular member 45 extends distally from the distal end of the proximal section of the agent delivery lumen 24 in the multilumen proximal shaft. First and second tubular members 44,45 are typically formed of thin-walled polymeric material such as polyimide, with an inner diameter of about 0.002 inch to about 0.006 inch, and a wall thickness of about 0.0005 inch to about 0.002 inch. In other embodiments, catheter shaft comprises an outer tubular member with first and second inner tubular members defining inflation lumen and agent delivery lumen, respectively, extending within the outer member and out the distal end thereof. The agent delivery lumen 24 extends to a location proximal to the distal end of the tubular member 13 and distal to the balloon. One or more agent delivery ports 25 are provided in a distal section of the agent delivery lumens, as discussed above in relation to the embodiment of FIG. 1. In other embodiments, one or more additional agent delivery lumens may be provided.

In the embodiment illustrated in FIG. 7, the frame 40 comprises longitudinally extending filaments or struts, such as wires, joined together at the proximal and distal ends thereof. In another embodiment, frame 40 is formed of high strength metal, such as stainless steel, nickel-titanium alloy, and titanium. However a variety of suitable materials can be used including rigid polymers. The filaments typically have a round transverse cross section, with a diameter of about 0.006 inch to about 0.016 inch, or a rectangular transverse cross section with a thickness of about 0.001 inch to about 0.006 inch and a width of about 0.006 inch to about 0.016 inch. Sheath 41 is similar to sheath 17 discussed in relation to the embodiment of FIG. 1, and is preferably a thin walled elastomeric tubular member. The tubular member 13 is illustrated in FIG. 7 in the expanded configuration. The frame 40 is radially collapsible to a low profile configuration with the sheath 41 in a folded or pleated compact configuration for advancement within the patient's blood vessel. Once in place at a desired location within the blood vessel, a restraining member which applies a radially compressive force, which holds the frame in the collapsed smaller diameter configuration, is removed so that the frame expands. The frame may be held in the collapsed smaller diameter configuration by a variety of suitable restraining members such as a delivery catheter or removable outer sheath. For example, in one embodiment, the frame is deformed into the smaller diameter configuration within the lumen of a delivery catheter 46, and then expanded in the blood vessel lumen by longitudinally displacing the frame out the distal end of the delivery catheter 46 to thereby remove the radially compressive force of the delivery catheter 46. Although not illustrated, a pull line similar to pull line 19 discussed above in relation to the embodiment of FIG. 1 may be provided to apply additional radially expanding force to the filaments to supplement their inherent spring force, and is preferably provided in the embodiments having a radially expandable member 14 comprising an inflatable balloon where inflation of the balloon creates a radially compressive force on the tubular member 13. In the embodiment illustrated in FIG. 7, balloon 42 is inflated into contact with the aorta wall 31 to an outer diameter which completely occludes the outer blood flow stream downstream of the renal arteries 32. Thus, the outer blood flow stream is directed into the branch vessels 32. However, the balloon may be configured to inflate to an outer diameter which does not completely occlude the downstream outer blood flow stream, as discussed above in relation to the embodiment of FIG. 3.

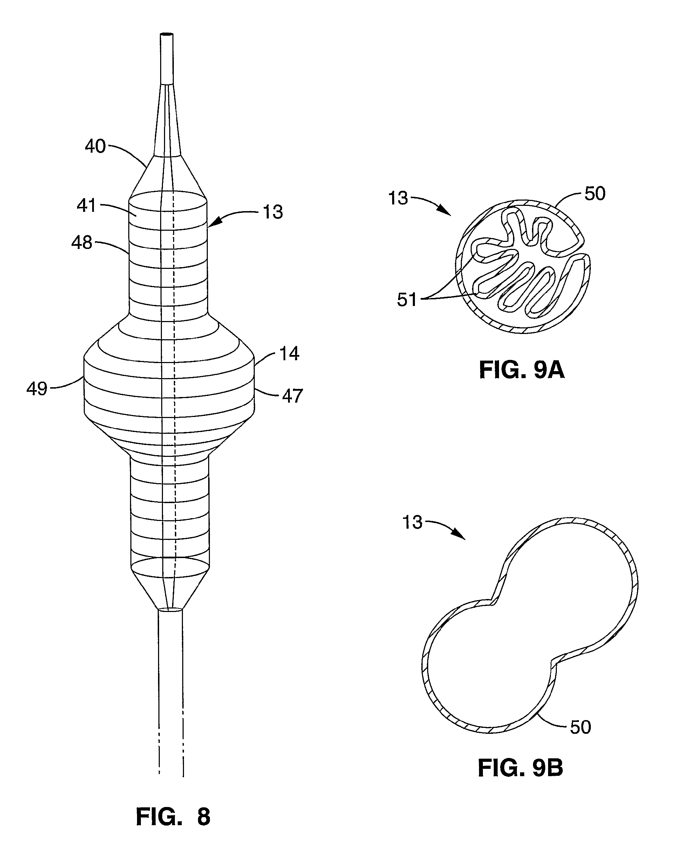

FIG. 8 illustrates another embodiment sharing certain similarities with the embodiment shown in FIG. 7 except that the balloon member 14 is replaced with a radially enlarged section 47 of the tubular member 13. Thus, the frame 40, with sheath 41 thereon, forming the tubular member 13 does not have a uniform outer diameter, but instead radially expands from a collapsed configuration to define a smaller diameter section 48 defining tubular member 13, and a larger diameter section 49 defining a larger radial expandable member 14.

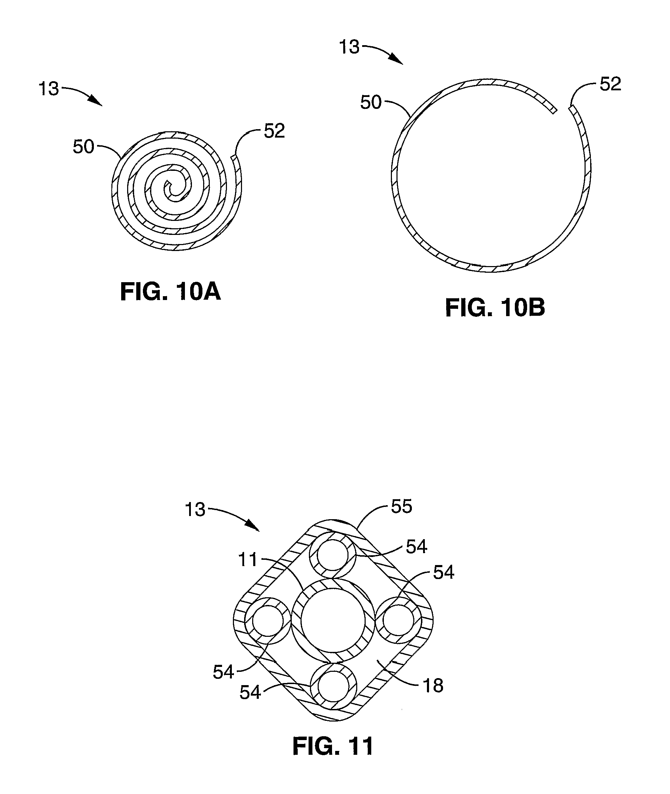

FIG. 9A and 9B and FIG. 10A and 10B illustrate transverse cross sectional views of another embodiment in which the tubular member 13 comprises a sheet 50 configured to unwind from a wound low profile to an unwound radially expanded configuration to thereby radially expand the interior passageway 18 of the tubular member 13. FIG. 9A illustrates an embodiment in which the sheet 50 has a section wound back and forth into a plurality of folds 51. A restraining member (not shown) such as an outer sheath or delivery catheter is removed so that the sheet 50 unfolds as illustrated in FIG. 9B. The sheet section configured to be folded is preferably a thinner walled or otherwise more flexible than the section of the sheet which is not folded. In another embodiment illustrated in FIG. 10A, the sheet 50 is wound around itself into a rolled-up configuration having a free edge 52 extending the length of the sheet 50, which unrolls to the radially expanded configuration illustrated in FIG. 10B. A variety of suitable unfurling or uncoiling configurations may be used in a tubular member which is radially expandable in accordance with the invention including a rolled awning-type mechanism, and the like.

FIG. 11 illustrates a transverse cross sectional view of another embodiment in which the tubular member 13 comprises a plurality of inflatable balloons 54 within an outer sheath 55. The balloons 54 can be inflated from a non-inflated low profile configuration to an inflated configuration. In the inflated configuration, inner passageway 18 is defined between the inflated balloons in part by the sheath 55. Preferably, three or more balloons 54 are provided to in part define the inner passageway 18. Balloons 54 are preferably formed of a noncompliant material such as PET, or a compliant material such as polyethylene having reinforcing members such as wire members. Although four, cylindrical balloons 54 are illustrated in FIG. 11, it should be understood that a variety of suitable configurations may be used, including balloons having outer channels such as a spiraled balloon defining an outer spirally extending blood flow channel, similar in many respects to perfusion balloons for dilatation. An inflation lumen is provided in the catheter shaft 11 in fluid communication with balloons 54.

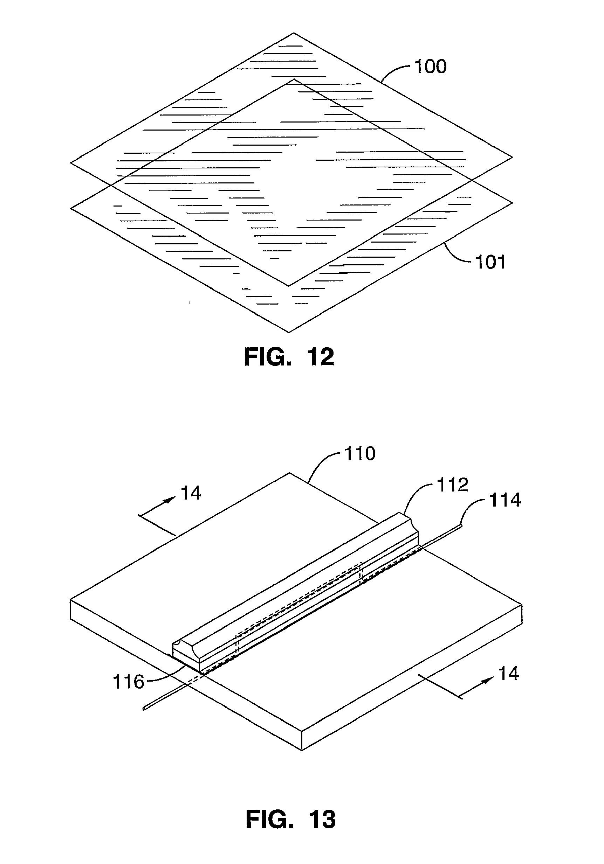

FIG. 12 through FIG. 21 illustrate a fabrication method according to one exemplary embodiment of the expandable tubular member. In FIG. 12, two sheets of a polymeric film, sheet 100 and sheet 101 are configured for seam welding. A variety of suitable materials can be used to form the sheets including polyolefins, low density polyethylene, polyurethane, polyamides, nylon, polyether block amide, polyethylene terephthalate, and other thermoplastics.

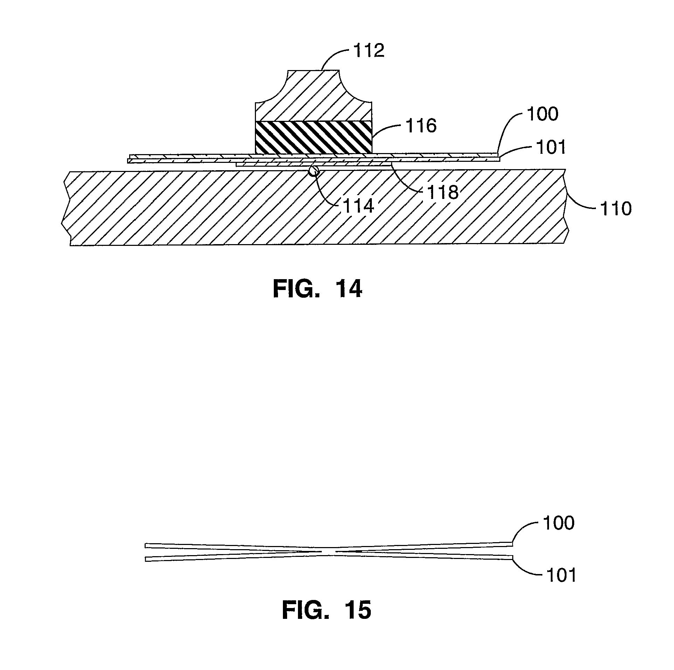

FIG. 13 and FIG. 14. illustrate a fixture for heat-fused welding sheets 100 and 101. Base 110 of the fixture provides a surface for press plate 112 to compress sheets 100 and 101 on a hot wire 114 in base 110. Press plate 112 has silicone rubber gasket 116 for contact with the film. PTFE coated sheet 118 is placed over hot wire 114 to prevent sheet 101 from sticking to hot wire 114.

FIG. 15 illustrates a cross section of a typical heat-fused weld of sheets 100 and 101.

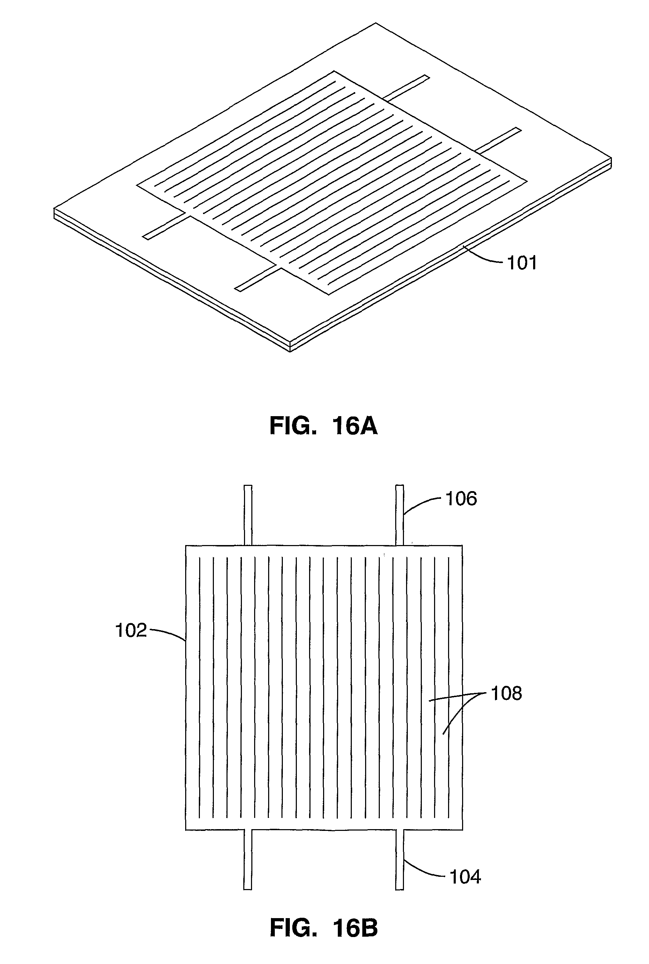

FIG. 16A shows a typical pattern of heat fused welds where sheets 100 and 101 are welded together with a pattern of seal lines to form inflatable member 102. Although these seal lines are shown in parallel, such a highly beneficial embodiment is not intended to be limiting to the various broad aspects of the present embodiment and other arrangements are contemplated to suit a particular need. The seal lines forming the wall chambers 108 do not extend to the proximal most and/or distal most end of the tubular member, so that the wall chambers are in fluid communication with one another. In the embodiments illustrated in FIG. 16A through FIG. 19B, the seal lines defining the wall chambers 108 of the tubular member 102 extend in parallel lines in inflatable member 102.

FIG. 16B illustrates inflatable member 102 following trimming. One or more inflation tabs 104 are formed and have fluid connection to the wall chambers 108 in inflatable member 102. One or more attachment tabs 106 are formed and are closed to the wall chambers 108. FIG. 16B illustrates one configuration of inflation tabs 104 and attachment tabs 106 but configurations may be formed and positioned at the proximal or distal ends of inflatable member 102.

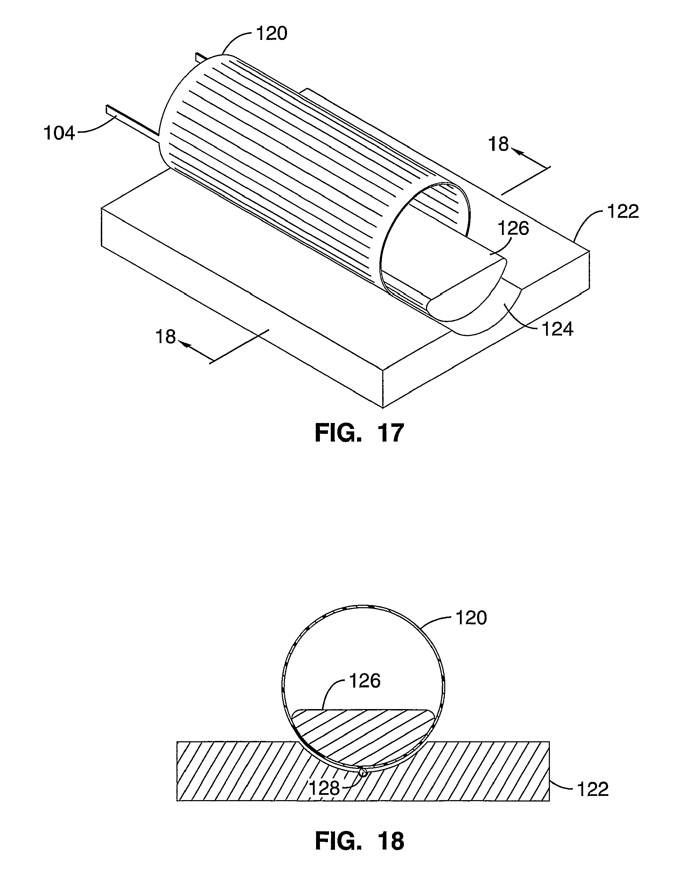

FIG. 17 is a perspective view and FIG. 18 is an end view of a wrapping and sealing fixture for inflatable member 102. Sealing fixture 122 has curved depression 124 and mandible 126 which conforms to curved depression 124. Inflatable member 102 is then wrapped into a cylindrical shape and the edges secured together with a heat-fused weld using hot wire 128 to form tubular member 120 which is collapsible and foldable into a compact configuration for advancement within the blood vessel. In the embodiments illustrated in FIG. 25 through FIG. 27, the seal lines defining the wall chambers of the tubular member 120 are parallel along the cylindrical wall of tubular member 120, but again while this is particularly beneficial, other configurations may be formed.

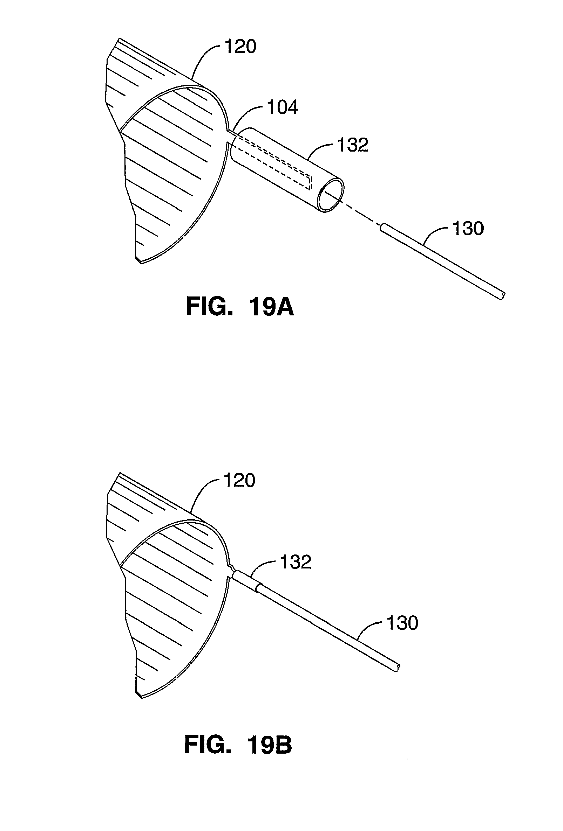

FIG. 19A and FIG. 19B illustrate the connection of inflation tube 130 to tubular member 120. Inflation tube 130 is inserted in inflation tabs 104 to be fluidly connected with the walled chambers 108 in tubular member 120. Adapting tube 132 is placed over inflation tab 104 and inflation tube 130 to provide a sealed connection. Adapting tube 132 can be a heat activated shrink tubing consisting of a heat-recoverable polyolefin, polyester, or other suitable material.

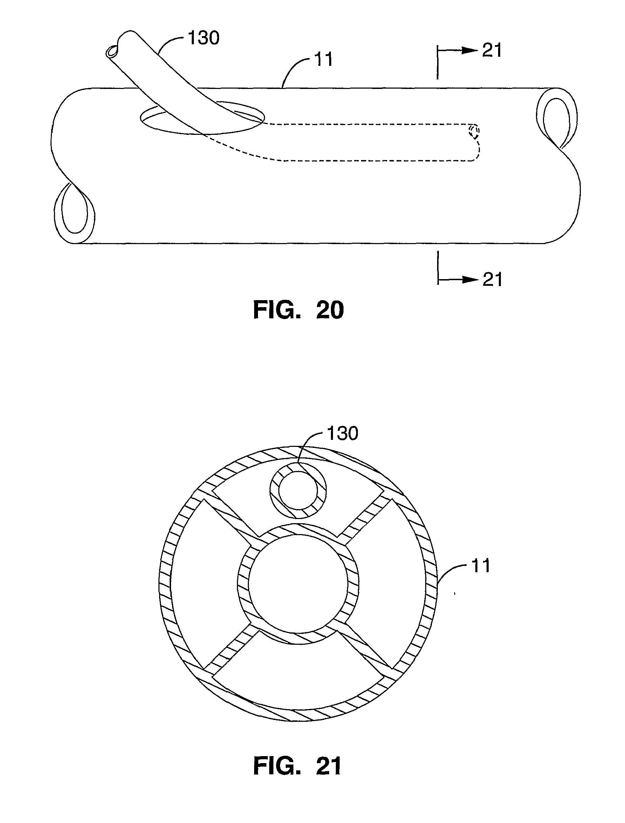

FIG. 20 is a side view and FIG. 21 is a transverse cross section of FIG. 20 illustrating the configuration of inflation tube 130 entering catheter shaft 11. Inflation tube 130 enters an inner lumen of catheter 11 where it is sealed with a potting compound such as epoxy or sealed with heat fusion.

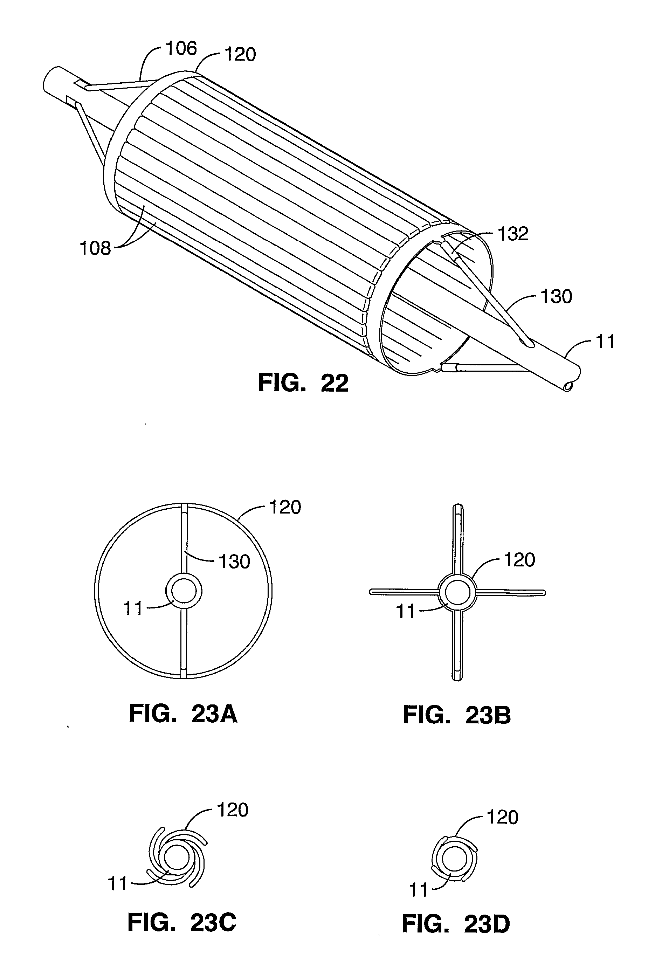

FIG. 22 illustrates an enlarged distal end of one embodiment having a tubular member 120 formed of a plurality of inflatable fluid-communicating wall chambers 108, in which one or more inflation tubes 130 extend from a port in the sidewall of shaft 11 in communication with an inflation lumen in shaft 11 to the distal and/or proximal end of the tubular member. The inflation tubes 130 are in fluid communication with the wall chambers of the tubular member 120, and are used for delivering inflation fluid into the wall chambers 108 to thereby inflate the tubular member 120. In the embodiment of FIG. 22, inflation tube 130 is secured to tubular member 120 by adapting tube 132 at the proximal end of tubular member 120. Attachment tabs 106 secure the distal end of tubular member 120 to the distal end of catheter shaft 11.

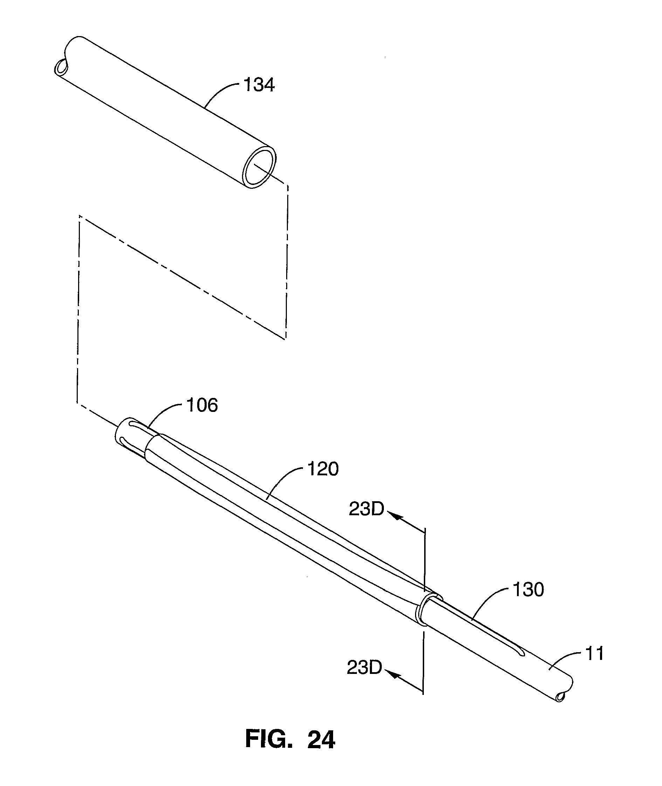

FIG. 23A through FIG. 24 illustrate a method by which tubular member 120 can be folded into a compact configuration for insertion. FIG. 23A is an end view with catheter shaft 11 in the center connected to tubular member 120 by inflation tubes 130. In FIG. 23B, tubular member 120 is folded into 4 wings about catheter shaft 11. In FIG. 23C, the wings of tubular member 120 are wrapped in pinwheel fashion and compacted in FIG. 23D. FIG. 24 illustrates compacted tubular member 120 enclosed in a tight sheath 134. The sheath may be formed of PTFE or other suitable material. During deployment, the sheath may be removed from the tubular member 120 by a pull wire (not shown) or other suitable method. The sheath may also be a delivery catheter.

FIG. 25 through FIG. 27 illustrate another embodiment wherein tubular member 120 comprises a plurality of wall chambers 108 joined together and attached to catheter shaft 11. The wall chambers 108 can be bonded together using a variety of suitable methods including an adhesive, heat fusion bonding, or solvent bonding such as with hexa-fluoro isopropanol (HFIP) for PET balloons. For ease of illustration, the radially expandable member 140 which is an inflatable balloon is shown as a transparent material. As best illustrated in FIG. 26 showing a transverse cross sectional view of the catheter shaft 11 taken along line 26-26, the multilumen shaft 11 defines an inflation lumen 21 in fluid communication with balloon 140 on an outer surface of the tubular member 120, (Similar to FIG. 5), and an agent delivery lumen 24 and a third inflation lumen 57 in fluid communication with the walled chambers 108. As best illustrated in FIG. 27 showing a transverse cross sectional view of the tubular member 120 taken along line 27-27, tubular member 120 is formed as a cylinder to thereby define the tubular member interior passageway 18. Catheter shaft 11 is inserted in a walled chamber 108. Agent delivery opening 27 in walled chamber 108 is adjacent to the shaft agent delivery port 25 and provides a pathway for agent delivery from the lumen 24 to exterior to the tubular member 120. The tubular member 120 can be deflated and compressed, folded, pleated or otherwise reduced in size for introduction and advancement within the patient's blood vessel as shown in FIG. 23A through FIG. 24. In one particular embodiment, the pressure required to inflate the balloon 140 is significantly lower than the pressure used to inflate the walled chambers 108 forming the tubular member 120, so that inflation of the balloon 140 does not deform the tubular member 120.

FIG. 28 illustrates the enlarged distal end of another embodiment having a tubular member 150 formed of a plurality of inflatable fluid-communicating wall chambers 108 in the shape of a cone. One or more inflation tubes 130 extend from a port in the sidewall of shaft 11 in communication with inflation lumen 57 (shown in FIG. 26) to the distal and/or proximal end of the tubular member 150. The inflation tubes 130 are in fluid communication with the wall chambers 108 of the tubular member 150, and are used for delivering inflation fluid into the wall chambers 108 to thereby inflate the tubular member 150. In the embodiment of FIG. 28, inflation tube 130 is secured to tubular member 150 by an adapting tube 132 at the proximal end of tubular member 150 as also shown in FIG. 22. One or more agent delivery tubes 136 extend from a port in the shaft 11 in fluid communication with agent delivery lumen 24 (shown in FIG. 26) and into wall chamber 108 of the tubular member 150. Agent delivery port 25 at the distal end of the agent delivery tube 136 extends to and in fluid communication with an agent delivery opening 27 in a wall defining wall chamber 108 of the tubular member 150 as shown in FIG. 27. The section of the agent delivery tube 136 located within wall chamber 108 of the tubular member 150 is illustrated in phantom in FIG. 28. Thus, one or more wall chambers 108 of the tubular member 150 can be used for agent delivery rather than inflation of tubular member 150.

In the embodiment illustrated in FIG. 28, tubular member 150 is conical. The conical tubular member 150 tapers from a large diameter proximal end 152 to a smaller diameter distal end 154, so that the large diameter distal end 152 of tubular member 150 replaces the radially expandable balloon member 140 shown in FIG. 25. Consequently, a separate radially expandable balloon member is not required.

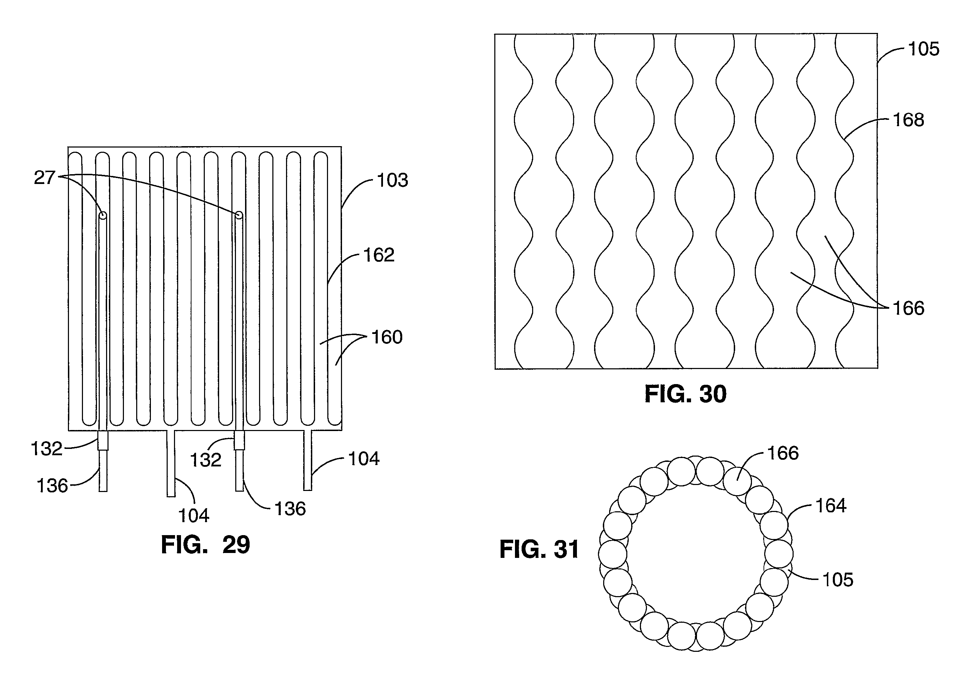

FIG. 29 illustrates an elevational view of inflatable member 103 for forming another embodiment of a tubular member (not shown) in which the wall chambers 160 are welded in a serpentine pattern 162. Wall chambers 160 are open at one end and are fluidly connected. Agent delivery tube 136 with agent delivery opening 27 is shown positioned within a walled chamber 160 and secured with adapting tube 132. Inflation tabs 104 are connected similar to the method described in FIG. 16B.

FIG. 30 illustrates an elevational view of inflatable member 105 for forming another embodiment of a tubular member 164, (shown in FIG. 31) in which the wall chambers 166 are defined by curvilinear seal lines 168 to form interleaved cells, so that a more complete occlusion is provided by the tubular member 164.

FIG. 31 illustrates a transverse cross-sectional view of an expanded tubular member 164 with wall chambers 166 formed from the curvilinear seal lines 168.

The invention has been discussed in terms of certain embodiments. One of skill in the art will recognize that various modifications may be made without departing from the scope of the invention. Although discussed primarily in terms of controlling blood flow to a branch vessel such as a renal artery of a blood vessel, it should be understood that the catheter of the invention could be used to deliver agent to branch vessels other than renal arteries, or to deliver to sites other than branch vessels, as for example where the catheter is used to deliver an agent to the wall defining the body lumen in which the catheter is positioned, such as a bile duct, ureter, and the like. Moreover, while certain features may be shown or discussed in relation to a particular embodiment, such individual features may be used on-the various other embodiments of the invention.

Although the description above contains many details, these should not be construed as limiting the scope of the invention but as merely providing illustrations of some of the presently preferred embodiments of this invention. Therefore, it will be appreciated that the scope of the present invention fully encompasses other embodiments which may become obvious to those skilled in the art, and that the scope of the present invention is accordingly to be limited by nothing other than the appended claims, in which reference to an element in the singular is not intended to mean "one and only one" unless explicitly so stated, but rather "one or more." All structural, chemical, and functional equivalents to the elements of the above-described preferred embodiment that are known to those of ordinary skill in the art are expressly incorporated herein by reference and are intended to be encompassed by the present claims. Moreover, it is not necessary for a device or method to address each and every problem sought to be solved by the present invention, for it to be encompassed by the present claims. Furthermore, no element, component, or method step in the present disclosure is intended to be dedicated to the public regardless of whether the element, component, or method step is explicitly recited in the claims. No claim element herein is to be construed under the provisions of 35 U.S.C. 112, sixth paragraph, unless the element is expressly recited using the phrase "means for."

The present application also comprises the following clauses:
1. A catheter for controlling blood flow to a branch vessel of a blood vessel, comprising:
   an elongated shaft;
   a tubular member on a distal section of the shaft having an interior passageway which is radially expandable within a blood vessel to separate blood flow through the blood vessel into an outer blood flow stream exterior to the tubular member and an inner blood flow stream within the interior passageway of the tubular member, and which is configured to extend within the blood vessel upstream and downstream of a branch vessel; and
   a radially expandable member on the tubular member, having an expanded configuration with an outer diameter larger than an outer diameter of the tubular member and which is configured to direct at least part of the blood flow in the outer blood flow stream into the branch vessel.
2. The catheter of clause 1 wherein the elongated shaft has at least one lumen therein in fluid communication with at least one agent delivery port in a distal section of the shaft, and wherein the radially expandable member is downstream of the agent delivery port.
3. The catheter of clause 2 wherein the tubular member has an upstream end located upstream of the agent delivery port.
4. The catheter of clause 2 wherein the tubular member has a distal end located distal to the agent delivery port.
5. The catheter of clause 2 wherein the agent delivery port is a lateral port in a side wall of the shaft.
6. The catheter of clause 1 wherein the radially expandible member comprises an inflatable balloon, and the shaft includes an inflation lumen and an inflation port in fluid communication with an interior of the balloon.
7. The catheter of clause 1 wherein the radially expandable member comprises a radially enlarged section of the tubular member.
8. The catheter of clause 1 wherein the tubular member comprises a braided tube having a sheath.
9. The catheter of clause 8 including a pull line having a distal end attached to the tubular member, and wherein the interior passageway of the tubular member is radially expanded by proximally retracting the pull line.
10. The catheter of clause 1 wherein the tubular member is self expanding.
11. The catheter of clause 10 wherein the tubular member comprises a radially collapsible frame having a sheath.
12. The catheter of clause 10 wherein the tubular member comprises a sheet configured to unwind from a wound low profile configuration to an unwound radially expanded configuration to thereby radially expand the interior passageway of the tubular member.
13. The catheter of clause 1 wherein the tubular member comprises a plurality of tubular balloons joined together, each tubular balloon being joined to adjacent tubular balloons along a length thereof to thereby define the tubular member interior passageway.
14. The catheter of clause 1 wherein the tubular member is conically shaped having a smaller diameter end and a larger diameter end.
15. The catheter of clause 14 wherein the radially expandable member is formed by the larger diameter end of the conical tubular member.
16. The catheter of clause 1 wherein the interior passageway of the tubular member has an expanded inner diameter of about 30 mm to about 130 mm.
17. The catheter of clause 1 wherein the interior passageway of the tubular member has an unexpanded inner diameter configured to expand to an expanded inner diameter, wherein the expanded inner diameter is about 1000% to about 6000% larger than the unexpanded inner diameter.
18. A method of controlling blood flow to a branch vessel of a blood vessel, comprising:
   providing a catheter comprising;
      an elongated shaft;
      a tubular member on the distal section of the shaft having an interior passageway which is radially expandable within a blood vessel, and which is configured to extend within the blood vessel upstream and downstream of a branch vessel; and
      a radially expandable member on the tubular member, having an expanded configuration with an outer diameter larger than an outer diameter of the tubular member and which is configured to decrease the blood flow in the outer blood flow stream downstream of the branch vessel; and
   advancing a distal portion of the catheter within the parent's descending aorta, so that an upstream end of the tubular member is upstream of the branch vessel and the radially expandable member is downstream of the branch vessel;
   expanding the tubular member to separate blood flow through the blood vessel into an outer blood flow stream exterior to the tubular member and an inner blood flow stream within the interior passageway of the tubular member; and
   expanding the radially expandable member to the expanded configuration to thereby decrease the blood flow in the outer-blood-flow stream downstream of the at least one renal artery.
19. The method of clause 18 including expanding the radially expandable member into contact with a wall of the blood vessel to occlude the outer blood flow stream downstream of the branch vessel.
20. The method of clause 18 including expanding the radially expandable member to an outer diameter which does not completely occlude the outer blood flow stream downstream of the branch vessel.
21. A catheter for delivering a therapeutic or diagnostic agent to a branch vessel of a blood vessel, comprising:
   an elongated shaft having at least one lumen therein in fluid communication with at least one agent delivery port in a distal section of the shaft;
   a tubular member on the distal section of the shaft having an interior passageway which is radially expandable within a blood vessel to separate blood flow through the blood vessel into an outer blood flow stream exterior to the tubular member and an inner blood flow stream within the interior passageway of the tubular member, and which is configured to extend within the blood vessel upstream and downstream of a branch vessel; and
   a radially expandable member on the tubular member, downstream of the shaft agent delivery port, having an expanded configuration with an outer diameter larger than an outer diameter of the tubular member and which is configured to decrease the blood flow in the outer blood flow stream downstream of the branch vessel.
22. A method of delivering a therapeutic or diagnostic agent to a patient's kidney, comprising:
   providing a catheter comprising;
      an elongated shaft having at least one lumen therein in fluid communication with at least one agent delivery port in a distal section of the shaft;
      a tubular member on the distal section of the shaft having an interior passageway which is radially expandable within a blood vessel, and which is configured to extend within the blood vessel upstream and downstream of a branch vessel; and
      a radially expandable member on the tubular member, downstream of the shaft agent delivery port, having an expanded configuration with an outer diameter larger-than an outer-diameter of the tubular member and which is configured to decrease the blood flow in the outer blood flow stream downstream of the branch vessel; and
   advancing a distal portion of the catheter within the patient's descending aorta so that the agent delivery port is upstream or adjacent to at least one renal artery of the patient, and the radially expandable member is downstream of the at least one renal artery;
   expanding the tubular member to separate blood flow through the descending aorta into an outer blood flow stream exterior to the tubular member and an inner blood flow stream within the interior passageway of the tubular member;
   expanding the radially expandable member to the expanded configuration to thereby decrease the blood flow in the outer blood flow stream downstream of the at least one renal artery; and
   flowing a therapeutic or diagnostic agent from the shaft lumen to the agent delivery port and into the outer blood flow stream in the aorta, to deliver the therapeutic or diagnostic agent to the at least one renal artery.
23. A method for enhancing renal function in a patient, comprising:
   diverting a portion of aortic blood flowing into a location within an abdominal aorta to flow along a diverted flow path substantially only into a plurality of renal arteries via a plurality of respective ostia having unique respective locations along the abdominal aorta wall;
   injecting a volume of fluid agent into the diverted flow path within the abdominal aorta at the location;
   wherein the injected fluid agent is delivered substantially only into the plurality of renal arteries via the diverted flow path into their respective ostia; and
   wherein the fluid agent being delivered into the plurality of renal arteries is adapted to enhance renal function.
24. The method of clause 23, wherein the local delivery of fluid agent into the plurality of renal arteries comprises:
   locally delivering a volume of diuretic fluid agent into the plurality of renal arteries.
25. The method of clause 24, wherein the local delivery of diuretic fluid agent into the plurality of renal arteries comprises:
   locally delivering a volume of Furosemide or an analog or derivative thereof into the plurality of renal arteries.
26. The method of clause 24, wherein the local delivery of diuretic fluid agent into the plurality of renal arteries comprises:
   locally delivering a volume of Thiazide or an analog or derivative thereof into the plurality of renal arteries.
27. The method of clause 23, wherein the local delivery of fluid agent into the plurality of renal arteries comprises:
   locally delivering a volume of vasopressor into the plurality of renal arteries.
28. The method of clause 27, wherein the local delivery of vasopressor into the plurality of renal arteries further comprises:
   locally delivering a volume of Dopamine or an analog or derivative thereof into the plurality of renal arteries.
29. The method of clause 23, wherein the local delivery of fluid agent into the plurality of renal arteries comprises:
   locally delivering a volume of vasodilator into the plurality of renal arteries.
30. The method of clause 23, wherein the local delivery of fluid agent into the plurality of renal arteries comprises:
   locally delivering a volume of a vasoactive fluid agent into the plurality of renal arteries.
31. The method of clause 23, wherein the local delivery of fluid agent into the plurality of renal arteries comprises:
   locally delivering a volume of Papaverine into the plurality of renal arteries.
32. The method of clause 23, wherein the local delivery of fluid agent into the plurality of renal arteries comprises:
   locally delivering a volume of an analog or derivative of Papaverine into the plurality of renal arteries.
33. The method of clause 23, wherein the local delivery of fluid agent into the plurality of renal arteries comprises:
   locally delivering a volume of a Calcium-channel blocker into the plurality of renal arteries.
34. The method of clause 23, wherein the local delivery of fluid agent into the plurality of renal arteries comprises:
   locally deleting a volume of Nifedipine into the plurality of renal arteries.
35. The method of clause 23, wherein the local delivery of fluid agent into the plurality of renal arteries comprises:
   locally delivering a volume of an analog or derivative of Nifedipine into the plurality of renal arteries.
36. The method of clause 23, wherein the local delivery of fluid agent into the plurality of renal arteries further comprises:
   locally delivering a volume of Verapamil into the plurality of renal arteries.
37. The method of clause 23, wherein the local delivery of fluid agent into the plurality of renal arteries further comprises:
   locally delivering a volume of an analog or derivative of Verapamil into the plurality of renal arteries.
38. The method of clause 23, wherein the local delivery of fluid agent into the plurality of renal arteries comprises:
   locally delivering a volume of Fenoldapam into the plurality of renal arteries.
39. The method of clause 23, wherein the local delivery of fluid agent into the plurality of renal arteries comprises:
   locally delivering a volume of an analog or derivative of Fenoldapam into the plurality of renal arteries.
40. The method of clause 23, wherein the local delivery of fluid agent into the plurality of renal arteries further comprises:
   locally delivering a volume of a dopamine DA₁ agonist into the plurality of renal arteries.
41. The method of clause 23, wherein the fluid agent is delivered into the plurality of renal arteries for between about 2 and about 72 hours.
42. The method of clause 41, wherein the fluid agent is delivered into the plurality of renal arteries for between about 4 and about 8 hours.
43. The method of clause 23, wherein the injected fluid agent is delivered substantially only into the plurality of renal arteries via their respective ostia simultaneously.
44. The method of clause 23, further comprising:
   allowing a second portion of aortic blood to flow along a second flow path downstream across the plurality of renal artery ostia and into downstream circulation while diverting the portion of aortic blood into diverted flow path into the renal arteries and also while delivering the fluid agent into the plurality of renal arteries via the diverted flow path.
45. A system for enhancing renal function in a patient, comprising:
   a delivery catheter with a proximal end portion and a distal end portion with a fluid delivery port;
   a flow diverter located along the distal end portion;
   a source of fluid agent;
   wherein the delivery catheter is adapted to couple to the source of fluid agent;
   wherein the delivery catheter is adapted to position the distal end portion at a location within an abdominal aorta associated with a plurality of renal artery ostia having unique respective positions along the abdominal aorta wall;
   wherein the flow diverter at the location is adapted to divert a portion of aortic blood flow along a diverted flow path substantially only into the plurality of renal arteries via their respective ostia;
   wherein the fluid delivery port is positioned relative to the flow diverter so as to inject a volume of the fluid agent from the source into the diverted flow path; and wherein the fluid agent is adapted to enhance renal function.
46. The system of clause 45, wherein the fluid agent comprises a diuretic.
47. The system of clause 46, wherein the diuretic comprises Furosemide or an analog or derivative thereof.
48. The system of clause 46, wherein the diuretic comprises Thiazide or an analog or derivative thereof.
49. The system of clause 45, wherein the fluid agent comprises a vasopressor.
50. The system of clause 49, wherein the vasopressor comprises Dopamine or an analog or derivative thereof.
51. The system of clause 45, wherein the fluid agent comprises a vasodilator.
52. The system of clause 45, wherein the fluid agent comprises a vasoactive agent.
53. The system of clause 45, wherein the fluid agent comprises Papaverine.
54. The system of clause 45, wherein the fluid agent comprises an analog or derivative of Papaverine.
55. The system of clause 45, wherein the fluid agent comprises a Calcium-channel blocker.
56. The system of clause 45, wherein the fluid agent comprises Nifedipine.
57. The system of clause 45, wherein the fluid agent comprises an analog or derivative of Nifedipine.
58. The system of clause 45, wherein the fluid agent comprises Verapamil.
59. The system of clause 45, wherein the fluid agent comprises an analog or derivative of Verapamil.
60. The system of clause 45, wherein the fluid agent comprises Fenoldapam.
61. The system of clause 45, wherein the fluid agent comprises an analog or derivative of Fenoldapam.
62. The system of clause 45, wherein the fluid agent composes a dopamine DA₁ agonist.
63. The system of clause 45, wherein the delivery catheter is further adapted to allow a second portion of aortic blood to flow downstream across the plurality of renal artery ostia and into downstream circulation while the flow diverter is adjusted to divert the portion of aortic blood flow along the diverted flow path and while the fluid agent is injected into the diverted flow path.
64. The system of clause 45, wherein the flow diverter comprises:
   an adjustable wall that is adjustable between a first position and a second position;
   wherein in the first position the flow diverter is adapted to be delivered to the location within the abdominal aorta; and
   wherein in the second position the adjustable wall is adapted to divert the portion of aortic blood flow along the diverted flow path.
65. The system of clause 64, wherein:
   the adjustable wall comprises a tubular member;
   the first position is characterized as a radially collapsed condition for the tubular member; and
   the second position is characterized as a radially expanded condition for the tubular member.
66. The system of clause 65, wherein:
   the tubular member comprises a conical shape.
67. The system of clause 65, wherein
   the tubular member comprises a frustroconical shape.
68. The system of clause 65, wherein:
   the tubular member comprises an inflatable member.
69. The system of clause 65, wherein:
   the tubular member is adapted to radially engage the abdominal aorta wall in the radially expanded condition at the location.
70. A system for treating a renal system in a patient, comprising:
   a renal drug delivery system; and
   a volume of fluid agent coupled to the renal drug delivery system;
   wherein the renal drug delivery system is adapted to deliver the volume of fluid agent bilaterally to each of two renal arteries simultaneously; and
   wherein the fluid agent comprises Papaverine, a Calcium-channel blocker, Nifedipine, Verapamil, Fenoldapam, or a dopamine DA₁ agonist.
71. A system for treating a renal system in a patient, comprising:
   a local renal drug delivery system;
   a volume of fluid agent coupled to the local renal drug delivery system;
   wherein the renal drug delivery system is adapted to deliver the volume of fluid agent bilaterally to each of two renal arteries simultaneously; and
   wherein the fluid agent comprises at least one of Papaverine, a Calcium-channel blocker, Nifedipine, Verapamil, Fenoldapam, a dopamine DA₁ agonist, or an analog or derivative thereof.
72. A method for treating a renal system in a patient, comprising:
   locally delivering a volume of fluid agent bilaterally into each of two renal arteries perfusing each of two kidneys, respectively;
   wherein the bi-lateral local delivery of the fluid agent is done substantially simultaneously; and
   wherein the fluid agent being bilaterally delivered into the renal arteries comprises Papaverine, a Calcium-channel blocker, Nifedipine, Verapamil, Fenoldapam, or a dopamine DA₁ agonist.
73. A method for treating a renal system in a patient, comprising:
   locally delivering a volume of fluid agent bilaterally into each of two renal arteries perfusing each of two kidneys, respectively;
   wherein the bi-lateral local delivery of the fluid agent is done substantially simultaneously; and
   wherein the fluid agent being bilaterally delivered into the renal arteries comprises at least one of Papaverine, a Calcium-channel blocker, Nifedipine, Verapamil, Fenoldapam, a dopamine DA₁ agonist, or an analog or derivative thereof.

## Claims

1. Femoldapam or an analogue or a derivative thereof for use in the treatment of acute renal failure or acute renal insufficiency in a patient, wherein said treatment comprises the administration of the medicament to at least one renal artery of the patient.

2. The compound of Claim 1, wherein said treatment comprises the administration of the medicament to both renal arteries of the patient.

3. The compound of Claim 2, wherein said treatment comprises the simultaneous administration of the medicament to both renal arteries of the patient.

4. The compound of Claim 1, wherein the acute renal failure is **characterized by** an abrupt decrease in the kidney's ability to excrete waste from the patient's blood.

5. The compound of Claim 1, wherein the acute renal failure is caused by sepsis.

6. The compound of Claim 1, wherein the acute renal failure is caused by hemorrhage.

7. The compound of Claim 1, wherein the acute renal failure is caused by surgery.

8. Use of femoldapam or an analogue or a derivative thereof for the manufacture of a medicament for the treatment of acute renal failure or acute renal insufficiency in a patient, wherein said treatment comprises the administration of the medicament to at least one renal artery of the patient.

9. The use according to Claim 8, wherein said treatment comprises the administration of the medicament to both renal arteries of the patient.

10. The use according to Claim 9, wherein said treatment comprises the simultaneous administration of the medicament to both renal arteries of the patient.

11. The use according to Claim 8, wherein the acute renal failure is **characterized by** an abrupt decrease in the kidney's ability to excrete waste from the patient's blood.

12. The use according to Claim 8, wherein the acute renal failure is caused by sepsis.

13. The use according to Claim 8, wherein the acute renal failure is caused by hemorrhage.

14. The use according to Claim 8, wherein the acute renal failure is caused by surgery.
